# EUROPÄISCHE PATENTSCHRIFT

(11) **EP 0 359 076 B1**
(45) Veröffentlichungstag und Bekanntmachung des Hinweises auf die Patenterteilung: **29.12.1993**
(21) Anmeldenummer: 89116252.1
(22) Anmeldetag: 02.09.1989
(51) Int. Cl.: C07D 215/56, C07D 471/04, C07D 498/06

(54) **PH-neutrale wässrige Lösungen von Chinolon-Carbonsäuren**
pH-neutral aqueous solutions of quinolone-carboxylic acids
Solution aqueuse à PH neutre d'acides quinolone carboxyliques

(30) Priorität: 16.09.1988 DE 3831514
(43) Veröffentlichungstag der Anmeldung: 21.03.1990
(73) Patentinhaber: BAYER AG, 51368 Leverkusen (DE)
(72) Erfinder: Naik, Arundev Harbhai Naik, D-4019 Monheim (DE); Voege, Herbert, Dr., D-5090 Leverkusen 3 (DE)

(56) Entgegenhaltungen:
- INORGANICA CHIMICA ACTA, Band 125, Nr. 1, 1986, Seiten 21-26, Elsevier Sequoia, CH; N.B. BEHRENS et al.: "Metal complexes of the antibiotic nalidixic acid"
- JOURNAL OF PARENTERAL SCIENCE & TECHNOLOGY, Band 40, Nr. 2, 1986, Seiten 70-72; C.H. SPURLOCK: "Increasing solubility of enoxacin and norfloxacin by means of salt formation"

## Beschreibung

Die vorliegende Erfindung betrifft pH-neutrale wässrige Lösungen von Chinoloncarbonsäuren, ihre Herstellung und ihre Verwendung als Bakterizide.

Bruteier werden mit Bakteriziden behandelt, um Ausfälle durch bakterielle Infektionen zu verhindern. Dazu wird eine Lösung eines Bakterizids, z.B. mit einer Injektionsspritze in die Eier gespritzt. Das Verfahren hat den Vorteil, daß jedes Ei eine definierte Wirkstoffmenge erhält. Das Verfahren ist allerdings arbeitsaufwendig.

Einfacher ist es, die Eier in einem Tauchbad zu behandeln. Damit der Wirkstoff besser durch die Schutzschicht der Eier dringen kann, werden die Eier entweder zunächst erwärmt (die Luftblase im Ei dehnt sich aus) und dann in eine kalte Wirkstofflösung getaucht oder die Eier werden in eine Wirkstofflösung getaucht und diese mit den Eiern unter Vakuum gesetzt. Bei beiden Verfahren wird etwas Wirkstofflösung durch die Eischale gesaugt. Allerdings schwankt die aufgenommene Menge Wirkstoff stark mit der Beschaffenheit der Schale.

Derartige Behandlungen von Bruteiern mit Bakteriziden und Antibiotika wie z.B. Gentamycin, Tetracyclin, Chloramphenicol waren bereits bekannt. Die Wirkung dieser Wirkstoffe, vor allem gegen resistente Bakterienstämme sowie gegen Mycoplasmen befriedigte in der Praxis nicht voll. Es war daher wünschenswert, die Behandlung der Bruteier mit Wirkstoffen vom Typ der Chinoloncarbonsäuren durchzuführen.

Chinoloncarbonsäuren sind in neutralem Wasser nur schwer löslich. Infolge ihres Betain-Charakters lassen sie sich in sauren oder alkalischen wässrigen Lösungen durch Salzbildung lösen. Solche Lösungen sind jedoch sehr (empfindlich gegen Schwankungen im pH-Bereich.

Zur Behandlung von Eiern können saure Lösungen nicht eingesetzt werden, weil sie die Eischale zu stark angreifen. Basische Lösung sind zur Behandlung von Eiern zwar grundsätzlich geeignet, haben jedoch den Nachteil, daß sie in geringer (für das Ei unschädlicher) Menge Magnesium und Calciumionen aus der Eischale herauslösen. Infolge der sehr geringen Löslichkeit des Magnesiumsalzes der Chinoloncarbonsäuren fällt dieses aus der Behandlungslösung aus. Die Behandlungslösung verarmt daher umso mehr an gelöstem Wirkstoff, je mehr Eier behandelt worden sind. Dann muß nicht nur verbrauchte Wirkstofflösung ersetzt werden, sondern zusätzlich Wirkstoff zur Aufrechterhaltung der Konzentration des gelösten Wirkstoffes zugesetzt werden (Aufkonzentration).

Ähnliche Probleme können überall dort auftreten, wo wässrige Lösungen von Chinoloncarbonsäuren eingesetzt werden. Es war daher wünschenswert, pH-neutrale, stabile wässrige Lösungen von Chinoloncarbonsäuren zur Verfügung zu haben, bei denen der Wirkstoff nicht in Form schwerlöslicher Salze ausfällt.

Aus *Journal of Parenteral Science and Technology*, *Bd. 40 (1986) S.70-72* ist bekannt, daß die Löslichkeit von Enoxacin und Norfloxacin mit organischen Säuren gesteigert werden kann.

Aus *Inorg.Chim.Act. 125 (1986) S.21-26* sind Metallkomplexe von Nalidixinsäure bekannt.

Keine dieser Literaturstellen gibt jedoch einen Hinweis darauf, wie die Löslichkeit von Chinoloncarbonsäuren im neutralen pH-Bereich erreicht werden kann.

Gegenstand der Erfindung sind pH-neutrale wässrige Lösungen von Chinoloncarbonsäuren, die erhalten werden, wenn man Chinoloncarbonsäuren mit wasserlöslichen Calciumsalzen in mindestens äquimolarem Verhältnis in Wasser umsetzt und den pH-Wert der Lösung anschließend auf einen Wert zwischen 6,5 und 7,5 einstellt.

Gegenstand der Erfindung ist ferner ein Verfahren zur Herstellung pH-neutraler wässriger Lösungen von Chinoloncarbonsäuren, das dadurch gekennzeichnet ist, daß man Chinoloncarbonsäuren mit wasserlöslichen Calciumsalzen in mindestens äquimolarem Verhältnis in Wasser umsetzt und den pH-Wert der Lösung anschließend auf einen Wert zwischen 6,5 und 7,5 einstellt.

Gegenstand der Erfindung ist ferner die Verwendung der hergestellten Lösungen, z.B. zur Behandlung von Eiern, als Trinkwasserformulierungen, Infusionen sowie in all den Fällen, in denen ph-neutrale wässrige Lösungen von Chinoloncarbonsäuren benötigt werden.

Es war überraschend, daß sich auf diese Weise stabile pH-neutrale wässrige Lösungen von Chinoloncarbonsäuren herstellen lassen. Zum einen sind die Magnesiumsalze von Chinoloncarbonsäuren sehr schwer wasserlöslich. Zum anderen bilden Chinoloncarbonsäuren mit stöchiometrischen Mengen Calciumsalz ebenfalls schwer lösliche Salze. Erst mit äquimolaren Mengen Calciumsalz und Chinoloncarbonsäure erhält man eine stabile wässrige Lösung.

Als Chinoloncarbonsäure kommen die Chinoloncarbonsäuren der allgemeinen Formel I in Frage 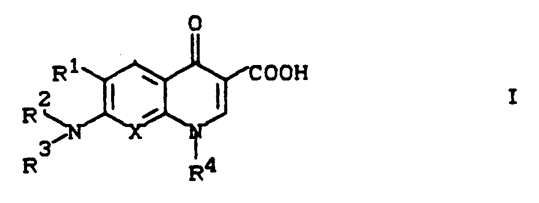
in welcher
- R¹: für Halogen, C₁₋₄-Alkyl, C₂₋₄-Alkenyl steht,
- R² und R³: für Wasserstoff, C₁₋₄-Alkyl stehen oder gemeinsam mit dem angrenzenden Stickstoffatom einen gegebenenfalls substituierten Morpholin-oder Piperazin-Ring bilden. Als Substituenten seien genannt C₁₋₄-Alkyl, C₁₋₄-Hydroxyalkyl.
- R⁴: für C₁₋₄-Alkyl oder C₃₋₆-Cycloalkyl steht, die gegebenenfalls substituiert sind.
- X: für -N= oder -CR⁵= steht,
- R⁵: für Wasserstoff, Halogen, OH oder C₁₋₄-Alkyl steht,
- R⁴ und R⁵: können gemeinsam mit den zwischen Ihnen liegenden C- und N-Atomen einen gesättigten 5-oder 6-Ring bilden, der noch weitere Heteroatome enthalten kann und gegebenenfalls substituiert ist.

Bevorzugt werden Verbindungen der Formel I verwendet, in der
- R¹: für Fluor, Chlor, Brom steht
- R² und R³: gemeinsam mit dem angrenzenden Stickstoffatom einen Morpholin- oder Piperazin-Ring bilden, die gegebenenfalls durch C₁₋₄-Alkyl, C₁₋₄-Hydroxyalkyl substituiert sind.
- R⁴: für C₁₋₄-Alkyl oder Cyclopropyl steht
- X: für -N= oder -CR⁵= steht,
- R⁵: für Wasserstoff, Fluor, Chlor oder Brom steht,
- R⁴ und R⁵: können daher gemeinsam mit den zwischen ihnen liegenden Atomen einen gesättigten 6-Ring bilden, der gegebenenfalls als weiteres Heteroatom O enthält und gegebenenfalls durch C₁₋₄-Alkyl substituiert ist.

Besonders bevorzugt werden Verbindungen der Formel I verwendet, in der
- R¹: für Fluor steht,
- R² und R³: gemeinsam mit dem angrenzenden Stickstoffatom ein gegebenenfalls durch C₁₋₄-Alkyl, C₁₋₄-Hydroxyalkyl substituierten Piperazin-Ring bilden,
- R⁴: für Ethyl oder Cyclopropyl steht,
- X: für N= oder -CR⁵= steht,
- R⁵: für Wasserstoff oder Fluor steht,
- R⁴ und R⁵: können gemeinsam mit den zwischen ihnen liegenden Atomen einen gesättigten 6-Ring bilden, der gegebenenfalls durch Methyl oder Ethyl substituiert ist.

Insbesondere seien genannt:
1-Cyclopropyl-6-fluor-1,4-dihydro-4-oxo-7-(1-piperazinyl-)chinolin-3-carbonsäure (Cyprofloxacin),
1-Cyclopropyl-6-fluor-1,4-dihydro-4-oxo-7-(4-ethyl-1-piperazinyl-)chinolin-3-carbonsäure (Enrofloxacin).
1-Ethyl-6-fluor-1,4-dihydro-4-oxo7-(1-piperazinyl)chinolin-3-carbonsäure,
1-Ethyl-6-fluor-1,4-dihydro-4-oxo-7-(1-piperazinyl)-1,8-naphthydrin-3-carbonsäure,
9-Fluor-3-methyl-10-(4-methyl-1-piperazinyl)-7-oxo-2,3-dihydro-7,4-pyrido[1,2,3-de]1,4-benzoxazin-6-carbonsäure.

Als wasserlösliche Calciumsalze kommen in Frage: Calciumacetat, Calciumchlorid, Calciumbromid, Calciumnitrat, Calciumphosphate bzw. deren Hydrate. Insbesondere sei Calciumchlorid bzw. sein Hexahydrat genannt.

Die Bildung der Calciumsalze kann auch mit Calciumhydroxid oder Calciumoxid durchgeführt werden.

Als Lösungsmittel für die erfindungsgemäßen Zubereitungen dient Wasser. Gegebenenfalls können auch Gemische von Wasser mit anderen Lösungsmitteln zum Einsatz kommen. Zu den Lösungsmitteln zählen: Alkohole wie ein- oder mehrwertige primäre sekundäre oder tertiäre Alkanole wie z.B. Ethanol, Butanol, Benzylalkohol, Glykol, Glycerin, Propylenglykol, sowie N-Methylpyrrolidon.

Die Konzentration der neben Wasser eingesetzten Lösungsmittel in den erfindungsgemäßen Zubereitungen liegt bei 1 bis 30 %, bevorzugt zwischen 1 bis 10 %, ganz besonders bevorzugt zwischen 1 bis 3 %.

Die erfindungsgemäßen Zubereitungen können mit üblichen Hilfsstoffen versetzt sein. Als solche kommen nichttoxische pharmazeutische Stoffe wie Verdünnungsmittel, Resorptionsbeschleuniger, Resorptionshemmer, Kristallisationsverzögerer, Komplexiermittel, Antioxidantien, Konservierungsmittel, Protonisierungsmittel.

Besonders bevorzugt seien genannt: Konservierungsmittel wie z.B. p-Hydroxy-benzoesäureester, Benzylalkohol oder Phenole, Antioxidantien wie z.B. Natrium-meta-bisulfit oder Natriumsulfit, Komplexiermittel wie Natriumsalze der Aethylendiamintetraessigsäure, Kristalllsationsverzögerer wie Polyvinylpyrrolidon.

Die Konzentration der Hilfsstoffe in den erfindungsgemäßen Zubereitungen liegt bei 0,1 bis 10 %., bevorzugt bei 1 bis 2 %.

Die Verbindungen der Formel I liegen in den erfindungsgemäßen Zubereitungen in Konzentrationen von 0,1 bis 30 %, bevorzugt je nach Art der Anwendung zwischen 0,5 bis 10 % oder 0,2 bis 2 % oder 10 bis 30 %. Besonders bevorzugt sind Lösungen mit 0,5 bis 10 % Verbindung der Formel I. Die Calciumsalze werden in dazu 1 bis 5fach äquimolarer Menge eingesetzt. Bevorzugt in etwa äquimolarer Menge.

Der pH-Wert der erfindungsgemäßen Zubereitungen liegt zwischen 6,5 und 7,5, bevorzugt bei 7.

Zur Herstellung der erfindungsgemäßen Zubereitungen kann man die Verbindungen der Formel I in Wasser in Lösung bringen und in dieser Lösung die notwendige Menge Calciumsalz als solches oder in Form seiner wässrigen Lösung einfügen. Anschließend wird die erhaltene Lösung mit Säure neutralisiert. In dieser Weise kann man sowohl gebrauchsfertige Lösungen der aktiven Substanz, abgefüllt in geeignete Behälter, zum Beispiel in Ampullen, Injektions- oder Infusionsflaschen, als auch zur Herstellung solcher für Lösungen geeignete Vorprodukte, z.B. Konzentrate, herstellen.

Die erfindungsgemäßen Lösungen sollen ebenso wie die Ihnen zugrunde liegenden Verbindungen der Formel I als Arzneimittel zur Bekämpfung bakterieller Infektionen verwendet werden. Besonders geeignet sind die Lösungen zur Behandlung von Eiern, z.B. gegen Bakterien- oder Mycoplasmeninfektionen.

### Beispiel 1

| 10%ige Zusammensetzung | |
|---|---|
| Enrofloxacin | 10,00 g |
| Kaliumhydroxid, 85,3 % | 1,80 g |
| Calciumchlorid, 6H₂O | 27,30 g |
| Salzsäure 1N bis pH 7,0 | ca. 1,00 g |
| Benzylalkohol | 1,00 g |
| Wasser | 100,00 ml |

Kaliumhydroxid wurde in Wasser gelöst anschließend der Wirkstoff Enrofloxazin unter Rühren zugegeben, gelöst und danach Calciumchlorid eingerührt. Dabei entstand eine Ausfällung. Es wurde unter Rühren solange Salzsäure zugegeben, bis eine klare Lösung von pH 7 entstand. Anschließend wurde mit Wasser bis 100 ml aufgefüllt.

Ein Teil dieser Lösung wurde mit 99 Teilen 5fach Standard WHO-Wasser verdünnt und zeigte über 25 Tage keine Ausfällungen.

### Beispiel 2

| | |
|---|---|
| 10 %ige Enrofloxacinlösung in wässriger Kaliumhydroxid-Lösung von pH11 mit 1 % Benzylalkohol | 50 ml |
| 55%ige wässrige Calciumchlorid 6H₂O-Lösung | 25 ml |
| 10%ige Essigsäure-Lösung | ca. 6,2 - 8,0 g |
| Wasser | ad 100 ml |

Zu der Enrofloxacin-Lösung wurde Calciumchlorid-Lösung unter Rühren zugegeben. Die entstehende Ausfällung wurde durch Zugabe von Essigsäure unter weiterem Rühren gelöst. Der pH-Wert wurde durch Zugabe von Essigsäure kontrolliert. Drei Formulierungen bei pH 6,7, pH 7, pH 7,2 wurden zubereitet.

### Stabilität der Formulierungen

Die drei Formulierungen (pH 6,7; 7,0; 7,2) waren während der Aufbewahrung bei 4°C und 50°C für zwei Monate stabil geblieben.

### Stabilität der Verdünnung

Die Formulierung (pH 7,0) wurde 1 + 49 (= 1000 ppm) in entmineralisiertem Wasser verdünnt und mit Zusatz von 20, 40, 60, 80 und 100 ppm Mg⁺⁺ über 26 Tage beobachtet, ohne daß Ausfällungen festgestellt wurden.

## Patentansprüche

1. PH-neutrale wässrige Lösungen von Chinoloncarbonsäuren der allgemeinen Formel I 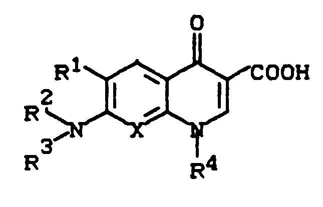 in welcher
R¹ für Halogen, C₁₋₄-Alkyl, C₂₋₄-Alkenyl steht,
R² und R³ für Wasserstoff, C₁₋₄-Alkyl stehen oder gemeinsam mit dem angrenzenden Stickstoffatom einen gegebenenfalls substituierten Morpholin-oder Piperazin-Ring bilden. Als Substituenten seien genannt C₁₋₄-Alkyl, C₁₋₄-Hydroxyalkyl.
R⁴ für C₁₋₄-Alkyl oder C₃₋₆-Ccloalkyl steht, die gegebenenfalls substituiert sind.
X für -N= oder -CR⁵= steht,
R⁵ für Wasserstoff, Halogen, OH oder C₁₋₄-Alkyl steht,
R⁴ und R⁵ können gemeinsam mit den zwischen Ihnen liegenden C- und N-Atomen einen gesattigten 5-oder 6-Ring bilden, der noch weitere Heteroatome enthalten kann und gegebenenfalls substituiert ist,
dadurch gekennzeichnet, daß man die Chinoloncarbonsäuren mit wasserlöslichen Calciumverbindungen in mindestens äquimolarem Verhältnis in Wasser umsetzt und den pH-Wert der Lösung anschließend auf einen Wert zwischen 6,5 und 7,5 einstellt.

2. Verfahren zur Herstellung pH-neutraler wässriger Lösungen von Chinoloncarbonsäuren der Formel I gemäß Anspruch 1, dadurch gekennzeichnet, daß man die Chinoloncarbonsäuren mit wasserlöslichen Calciumverbindungen in mindestens äquimolarem Verhältnis in Wasser umsetzt und den pH-Wert der Lösung anschließend auf einen Wert zwischen 6,5 und 7,5 einstellt.

3. Verwendung von pH-neutralen wässrigen Lösungen von Chinoloncarbonsäuren der Formel I gemäß Anspruch 1 zur Behandlung von Eiern und Trinkwasser.

4. pH-neutrale wässrige Lösungen gemäß Anspruch 1, dadurch gekennzeichnet, daß sie Enrofloxacin enthalten.

5. pH-neutrale wässrige Lösungen gemäß Anspruch 4, dadurch gekennzeichnet, daß sie pro Mol. Enrofloxacin 1 - 5 Mol. wasserlösliche Calciumsalze enthalten.

## Claims

1. pH-Neutral agueous solutions of quinolonecarboxylic acids of the general formula I 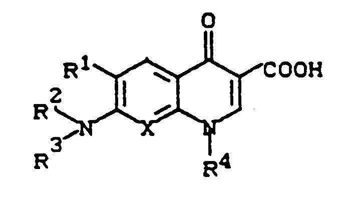 in which
R¹ represents halogen, C₁₋₄-alkyl, C₂₋₄-alkenyl,
R² and R³ represent hydrogen, C₁₋₄-alkyl, or together with the adjacent nitrogen atom form an optionally substituted morpholine or piperazine ring. Substituents which may be mentioned are C₁₋₄-alkyl, C₁₋₄-hydroxyalkyl.
R⁴ represents C₁₋₄-alkyl or C₃₋₆-cycloalkyl, which are optionally substituted.
X represents -N= or -CR⁵=,
R⁵ represents hydrogen, halogen, OH or C₁₋₄-alkyl,
R⁴ and R⁵, together with the C and N atoms positioned between them, can form a saturated 5- or 6-ring which can contain other hetero atoms and which is optionally substituted,
characterized in that the quinolonecarboxylic acids are reacted in water with water-soluble calcium compounds in at least an equimolar ratio and the pH of the solution is subsequently adjusted to a value of between 6.5 and 7.5.

2. Process for the preparation of pH-neutral aqueous solutions of quinolonecarboxylic acids of the formula I according to Claim 1, characterized in that the quinolonecarboxylic acids are reacted in water with water-soluble calcium compounds in at least an equimolar ratio and the pH of the solution is subsequently adjusted to a value of between 6.5 and 7.5.

3. Use of pH-neutral aqueous solutions of quinolonecarboxylic acids of the formula I according to Claim 1 for treating eggs and drinking water.

4. pH-Neutral aqueous solutions according to Claim 1, characterized in that they contain enrofloxazin.

5. pH-Neutral aqueous solutions according to Claim 4, characterized in that they contain 1 to 5 mol of water-soluble calcium salts per mole of enrofloxazin.

## Revendications

1. Solutions aqueuses à pH neutre d'acides quinolone-carboxyliques de formule générale I : 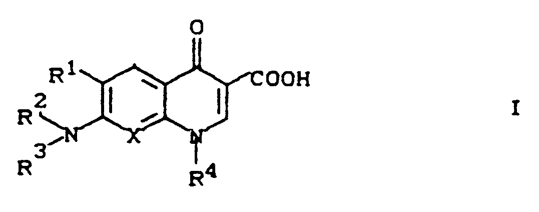 dans laquelle
R¹ représente un halogène, un groupe alkyle en C₁-C₄, alcényle en C₂-C₄,
R² et R³ représentent chacun l'hydrogène, un groupe alkyle en C₁-C₄, ou forment ensemble et avec l'atome d'azote voisin un cycle morpholine ou pipérazine éventuellement substitué, les substituants étant par exemple des groupes alkyle en C₁-C₄, hydroxyalkyle en C₁-C₄,
R⁴ représente un groupe alkyle en C₁-C₄ ou cycloalkyle en C₃-C₆, éventuellement substitués,
X représente -N= ou -CR⁵=,
R⁵ représente l'hydrogène, un halogène, un groupe OH ou alkyle en C₁-C₄,
R⁴ et R⁵ peuvent former, ensemble et avec les atomes de C et N placés entre eux, un cycle saturé à 5 ou 6 chaînons qui peut encore contenir d'autres hétéroatomes et est éventuellement substitué,
caractérisées en ce que l'on fait réagir les acides quinolone-carboxyliques dans l'eau avec des composés du calcium solubles dans l'eau en proportions au moins équimoléculaires puis on règle le pH de la solution à un niveau de 6,5 à 7,5.

2. Procédé de préparation des solutions aqueuses à pH neutre d'acides quinolone-carboxyliques de formule I de la revendication 1, caractérisé en ce que l'on fait réagir les acides quinolone-carboxyliques dans l'eau avec des composés du calcium solubles dans l'eau à un rapport au moins équimoléculaire et on règle ensuite le pH de la solution à un niveau de 6,5 à 7,5.

3. Utilisation des solutions aqueuses à pH neutre d'acides quinolone-carboxyliques de formule I de la revendication 1 pour le traitement des oeufs et de l'eau potable.

4. Solutions aqueuses à pH neutre selon la revendication 1, caractérisées en ce qu'elles contiennent de l'Enrofloxacin.

5. Solutions aqueuses à pH neutre selon la revendication 4, caractérisées en ce qu'elles contiennent de 1 à 5 mol de sels de calcium solubles dans l'eau par mole d'Enrofloxacin.
